Europäisches Patentamt

**European Patent Office**

**Office européen des brevets**

(11) Numéro de publication: **0 006 050**
**A1**

# DEMANDE DE BREVET EUROPEEN

(21) Numéro de dépôt: **79400324.4**

(22) Date de dépôt: **23.05.79**

(51) Int. Cl.²: **A 61 K 35/78**

(30) Priorité: **31.05.78 GB 2561878**

(43) Date de publication de la demande:
**12.12.79 Bulletin 79/25**

(84) Etats Contractants Désignés:
**AT BE CH DE FR GB IT LU NL SE**

(71) Demandeur: **Société Anonyme dite: LABORATOIRES DEBAT**
**60 rue de Monceau**
**F-75008 Paris(FR)**

(72) Inventeur: **Debat, Jacques**
**3 rue du Pierrier**
**F-92210 Saint Cloud(FR)**

(72) Inventeur: **Lemoine, Jean**
**7 sente de la Portaille**
**F-92380 Garches(FR)**

(72) Inventeur: **Lier, Françoise née Gabillault**
**7 rue Hector Berlioz Domaine de la Bataille**
**F-78370 Plaisir(FR)**

(74) Mandataire: **Clisci, Serge et al,**
**CABINET BEAU DE LOMENIE 55 rue d'Amsterdam**
**F-75008 Paris(FR)**

(54) Extrait de Stephania cepharantha, son procédé de préparation et son application en thérapeutique.

(57) La présente invention concerne un extrait de tiges de *Stephania cepharantha* préparé par traitement desdites tiges avec au moins un solvant choisi parmi l'ensemble constitué par l'eau, les alcools, les cétones, les esters, les éthers, les hydrocarbures, les hydrocarbures halogénés et leurs mélanges.

Cet extrait est utile en thérapeutique notamment en tant qu'agent bactériostatique, anti-inflammatoire et antalgique.

EP 0 006 050 A1

Extrait de Stephania cepharantha, son procédé de préparation et son application en thérapeutique.

La présente invention a trait à un extrait de Stephania cepharantha, à son procédé de préparation et à l'utilisation dudit extrait en thérapeutique, notamment en tant qu'agent bactériostatique, antalgique et anti-inflammatoire.

On sait que la plante Stephania cepharantha appartient à la famille des Menispermacées et pousse principalement en Chine et au Japon. Cette plante a été décrite par R.R. PARIS et H. MOYSE dans "Matière Médicale" vol. 2, page 178 (Masson ed., Paris, 1967).

On sait également, en particulier de la publication de R.R. PARIS et H. MOYSE, que des extraits de racines de Stephania Cepharantha, préparés par extraction avec un mélange eau-alcool /¯tel que le mélange eau-éthanol (50 : 50) v/v_7 à 60 - 100°C, ont été proposés en médecine populaire dans le traitement de la tuberculose et de la lèpre.

Il a été à présent trouvé que l'extrait de tiges de Stephania cepharantha est (i) différent des extraits de racines antérieurement connues, et (ii) utile en thérapeutique, en particulier dans le traitement des maladies infectieuses en raison de ses propriétés bactériostatique , antalgique et anti-inflammatoire.

La présente demande a donc pour objet de préconiser un procédé d'extraction de tiges de Stephania cepharantha en vue d'obtenir un nouvel extrait qui est utile dans le traitement des êtres humains souffrant en particulier de maladies infectieuses.

Le procédé selon l'invention est caractérisé en ce que les tiges de la plante sont extraites avec au moins un solvant. Le produit ainsi obtenu est purifié, si nécessaire, et recueilli selon une méthode connue en soi.

L'extraction peut être effectuée en utilisant pour un litre de solvant, 30 à 150 g de tiges sèches et broyées. Parmi les solvants utilisables à cet effet, on peut notamment mentionner l'eau, les alcools (tels que les méthanol, éthanol, propanol et isopropanol); les cétones (telles que les acétone, méthyléthylcétone et méthylpropyl-cétone), les éthers (tels que les éther diméthylique, éther diéthylique et éther diisopropylique), les esters (tels que l'acétate d'éthyle), les hydrocarbures (tels que les pentane, hexane, cyclopentane, cyclohexane, éther de pétrole et benzène), les hydrocarbures halogénés (tels que les chloroforme et chlorure de méthylène) et leurs mélanges.

Le meilleur mode pour mettre en oeuvre le procédé objet de l'invention consiste à extraire les tiges de Stephania cepharantha avec le chloroforme, le méthanol ou le mélange méthanol-eau (75 : 25) v/v.

L'extrait qui est obtenu par extraction avec un solvant puis évaporation à sec sous pression réduite, convient généralement des composants de nature alcaloïdique et des composants de nature non alcaloïdique. Les techniques préférées pour recueillir les substances alcaloïdiques (également désignées par le terme "alcaloï-des" ci-après) sont données dans les exemples 2 et 3, la technique de l'exemple 3 permettant d'isoler en même temps les substances de nature alcaloïdique et les substances de nature non alcaloïdique.

EXEMPLE 1 :

Préparation de l'extrait total.

140 g de tiges broyées et sèches de Stephania cepha-rantha sont extraits avec 3 litres de méthanol dans un appareil de Soxhlet pendant 4 heures. L'insoluble est écarté et la solution méthanolique est évaporée à sec sous pression réduite pour donner un produit sec qui est repris dans la quantité minimale d'eau puis lyophilisé. On obtient 85 g d'extrait total (qui est codé AJ-01). Le rendement est de 42,5 % en poids par rapport aux tiges de départ.

EXEMPLE 2 :

Extraction des substances de nature alcaloïdique.

a) <u>Extraction</u>

1,2 kg de tiges de <u>Stephania cepharantha</u> séchées (à l'étuve à 37°C) et broyées sont traités avec 1,2 litres de $NH_4OH$ (contenant 150 g/l de $NH_3$) et extraits avec 9 litres de $CHCl_3$ dans un appareil de Soxhlet. La solution chloroformique qui est recueillie est concentrée jusqu'à 3 litres sous pression réduite et extraite avec de l'eau acidulée (5 fois 500 ml d'eau contenant 20 g/l d'acide citrique). Après addition de $NH_4OH$ jusqu'à pH 8, les bases libres contenues dans là solution sont extraites au chloroforme (5 fois 200 ml). La phase organique est séchée sur sulfate de sodium anhydre et évaporée à sec sous pression réduite pour donner 11,76 g de produit sec appelé extrait total alcaloïdique. Rendement 9,8 °/₀₀ en poids par rapport au poids des tiges de départ.

b) <u>Purification</u>

L'extrait alcaloïdique total est soumis à une extraction avec du méthanol dans un appareil de Soxhlet. La solution méthanolique est évaporée à sec sous pression réduite. Le résidu d'évaporation est traité avec de l'eau contenant 20 g/l d'acide citrique et l'insoluble est écarté par filtration. On ajoute $NH_4OH$ à la phase aqueuse jusqu'à pH 8 et on lave avec du chloroforme (3 fois 200 ml). Après acidification (avec de l'acide citrique) les alcaloïdes quaternisés sont précipités au moyen du réactif de Mayer pour donner, après filtration et séchage, des iodomercurates d'alcaloïdes (51 g) qui sont transformés en chlorure d'alcaloïdes quaternisés par chromatographie avec une résine échangeuse d'ions (Amberlite IRA 400 sous la forme chlorure). Après élution et lyophilisation, on obtient les alcaloïdes.

EXEMPLE 3 :

Extraction des substances de nature alcaloïdique et des substances non alcaloïdiques.

100 g de tiges de <u>Stephania capharantha</u> séchées à l'étuve à 37°C et broyées sont extraits avec un litre du mélange méthanol – eau (75 : 25) v/v à l'ébullition. Après filtration de l'insoluble, on concentre le filtrat jusqu'à un volume de 250 cm3 sous pression réduite. La phase aqueuse qui est obtenue est filtrée et chromatographiée sur une résine non ionique (300 ml de résine Amberlite XAD₂).

4                    0006050

Le filtrat , qui renferme les produits non adsorbés, est lyophilisé . Le produit ainsi obtenu contient les substances de nature non alcaloïdique. L'élution des produits adsorbés au moyen d'un mélange éthanol - eau (90 : 10) v/v donne, après concentration sous pression réduite puis lyophilisation, un extrait contenant les substances de nature alcaloïdique.

Les extraits de <u>Stephania cepharantha</u> selon l'invention ont été testés en ce qui concerne leurs propriétés pharmacologiques. Les essais relatifs à l'extrait AJ-01 (obtenu à l'exemple 1) ont été résumés ci-après.

1) <u>Toxicité</u>

AJ-01 administré à des animaux par voie i.v. et i.p. en solution dans du sérum physiologique (eau contenant 9 g/1 de NaCl) est bien toléré. La DL-0 (dose maximale non mortelle) par voie i.v. et i.p. chez la souris est supérieure à 500 mg/kg.

2) <u>Activité bactériostatique</u>

AJ-01 présente une activité bactériostatique vis-à-vis des bactéries gram (+) et gram (-). Par exemple, les CMI (concentration minimale inhibitrice) de AJ-01 sont respectivement de 2 mg/ml vis-à-vis du <u>Staphylococcus aureus</u> Londres et de 44 mg/ml sur une souche de <u>Proteus</u> (<u>Proteus</u> 1557 du Catalogue collection du "Centre International de Distribution de Souches et d'Information sur les Types Microbiens" de Lausanne).

3) <u>Activité anti-inflammatoire</u>

L'activité anti-inflammatoire de AJ-01 a été étudiée sur le rat femelle (de poids 100 g) selon le test de l'oedème à la carragéenine. Les résultats donnés dans le tableau I pour AJ-01 et la Phénylbutazone (produit de référence) montrent que AJ-01 inhibe de façon statistiquement significative le développement de l'oedème à la carragéenine à la dose de 100 mg/kg, l'action étant manimale 3 heures après l'administration.

4) <u>Activité antalgique</u>

L'étude a été effectuée selon la méthode de KOSTER par administration i.p. à des souris mâles de 0,2 ml d'une solution aqueuse d'acide citrique (30 g/1) 30 minutes après administration i.p. ou orale des produits (AJ-01 et aspirine en tant que produit de référence) à tester. Les résultats ont été donnés dans le tableau II dans lequel le nombre de "crampings" (contraction intermittente de l'abdomen avec torsion du tronc et extension des pattes postérieures) et la variation en pourcentage par rapport aux animaux témoins ont été indiqués.

Les résultats des essais pharmacologiques démontrent que les extraits de tiges de <u>Stephania cepharantha</u> selon l'invention tels que AJ-01 sont utiles dans le traitement des êtres humains souffrant de maladies infectieuses, d'algie et d'inflammation.

Selon l'invention, on préconise une composition thérapeutique caractérisée en ce qu'elle renferme, en association avec un excipient physiologiquement acceptable, une quantité pharmaceutique d'un extrait selon l'invention.

Une telle composition peut être administrée par voie orale sous forme de dragées, pillules, sirops et ampoules buvables, par voie locale sous la forme de crème et de gel ou d'aérosol, ou par voie injectable.

## TABLEAU I

### OEDEME A LA CARRAGEENINE

| Produit (dose) | Nombre d'animaux par lot | Mesure Plethysmographique du volume de l'oedème après administration de carragéenine et produits à tester à l'instant T (a) | | | | |
|---|---|---|---|---|---|---|
| | | T + 1 h | T + 2 h | T + 3 h | T + 4 h | T + 5 h |
| témoins | 10 | $4,3 \pm 1,3$ | $7,2 \pm 1,6$ | $10,6 \pm 2,1$ | $12,7 \pm 2,2$ | $13 \pm 1,3$ |
| Phénylbutazone (20 mg/kg) | 8 | $4,6 \pm 0,3$ (+ 7 %) | $3,8 \pm 0,7$ (- 48 %) | $5,2 \pm 1,5$ (- 51 %) | $7,2 \pm 1,6$ (-43 %) | $8,3 \pm 1,5$ (- 36 %) |
| AJ-01 (20 mg/kg) | 10 | $4,0 \pm 0,4$ (- 8 %) | $5,9 \pm 1,4$ (- 18 %) | $10,6 \pm 1,4$ (0 %) | $12,7 \pm 1,1$ (0 %) | $12,7 \pm 1,1$ (- 2 %) |
| AJ-01 (100 mg/kg) | 8 | $3,6 \pm 1,6$ (- 16 %) | $5,5 \pm 0,8$ (- 24 %) | $7,3 \pm 1,9$ (-31%) (b) | $10 \pm 1,2$ (- 21%)(b) | $11,5 \pm 1$ (- 12 %) |

Notes  (a) : La variation en pourcentage par rapport aux témoins est donnée entre parenthèses.

(b) : Statistiquement significatif ($p < 0,05$).

## TABLEAU II
### TEST A L'ACIDE ACETIQUE

| Produit (dose) | Nombre d'animaux | Nombre de "crampings" | variation par rapport aux témoins |
|---|---|---|---|
| témoins | 10 | $28,1 \pm 6,8$ | - |
| Aspirine 200 mg/kg p.o. | 10 | $17,7 \pm 5,0$ (a) | - 37 % |
| AJ-01 100 mg/kg i.p. | 10 | $25,0 \pm 7,2$ | - 11 % |
| AJ-01 250 mg/kg i.p. | 10 | $19,1 \pm 4,0$ (a) | - 32 % |

Note : (a)   Statistiquement significatif (p  0,05)

## REVENDICATIONS

1. Procédé de préparation d'un extrait de Stephania cepharantha utile en thérapeutique caractérisé en ce que l'on extrait les tiges de la plante, broyées et sèchées, avec au moins un solvant choisi parmi l'ensemble constitué par l'eau, les alcools, les cétones, les esters, les éthers, les hydrocarbures, les hydrocarbures halogénés et leurs mélanges.

2. Procédé de préparation d'un extrait de Stephania cepharantha utile en thérapeutique notamment en tant qu'agent bactériostatique, anti-inflammatoire et antalgique, caractérisé en ce que on extrait 30 à 150 g de tiges Stephania cepharantha broyées et séchées avec un litre de solvant choisi parmi l'ensemble constitué par l'eau, les alcools, les cétones, les esters, les éthers, les hydrocarbures, les hydrocarbures halogénés et leurs mélanges, puis soumet la solution ainsi obtenue à une évaporation à sec sous pression réduite..

3. Procédé selon la revendication 2, caractérisé en ce que le solvant est choisi parmi l'ensemble constitué par le chloroforme , le méthanol et le mélange méthanol - eau (75 : 25) v/v.

4. Extrait de tiges de Stephania cepharantha utile en thérapeutique caractérisé en ce qu'il est préparé selon le procédé de la revendication 1.

5. Extrait de tiges de Stephania cepharantha utile en thérapeutique caractérisé en ce qu'il est préparé selon le procédé de la revendication 2.

6. Extrait de tiges de Stephania cepharantha utile en thérapeutique caractérisé en ce qu'il est préparé selon le procédé de la revendication 3.

7. Composition thérapeutique caractérisée en ce qu'elle renferme, en association avec un excipient physiologiquement acceptable, un extrait de tiges de Stephania cepharantha.

# 0006050

**Office européen des brevets**

## RAPPORT DE RECHERCHE EUROPEENNE

Numéro de la demande

EP 79 40 0324

## DOCUMENTS CONSIDERES COMME PERTINENTS

| Catégorie | Citation du document avec Indication, en cas de besoin, des parties pertinentes | Revendica-tion concernée |
|---|---|---|
| | CHEMICAL ABSTRACTS, vol. 68, 11-3-1968, no. 11, réf. 49838x, page 4836 COLUMBUS OHIO (US) & Yakugaku Zasshi 87 (10) 1203-8 (1967) MASAO TOMITA et al. "Alkaloids of menispermaceous plants. CCXLV Alkaloids of Stephnaia cephalantha. 7. Structure of cepharamine"  * abrégé en entier *  — | 1,2 |
| A | CHEMICAL ABSTRACTS, vol. 47, 25-3-1953, no. 10, réf. 5011c, page 5011 COLUMBUS OHIO (US) & Japan. J. Exptl.Med. 22, 77-85 (1952) SHUJI HASEGAWA et al. "Effects of alkaloids on the production of plasma cells and of ribonucleic acid"  * abrégé en entier *  — | 1,7 |

**CLASSEMENT DE LA DEMANDE (Int. Cl.²)**

A 61 K 35/78

**DOMAINES TECHNIQUES RECHERCHES (Int. Cl.²)**

A 61 K 35/78

**CATEGORIE DES DOCUMENTS CITES**

X: particulièrement pertinent
A: arrière-plan technologique
O: divulgation non-écrite
P: document intercalaire
T: théorie ou principe à la base de l'invention
E: demande faisant interférence
D: document cité dans la demande
L: document cité pour d'autres raisons
&: membre de la même famille, document correspondant

Le présent rapport de recherche a été établi pour toutes les revendications

| Lieu de la recherche | Date d'achèvement de la recherche | Examinateur |
|---|---|---|
| La Haye | 24-08-1979 | REMPP |

OEB Form 1503.1  06.78